# EUROPEAN PATENT SPECIFICATION

(11) **EP 4 019 646 B1**
(45) Date of publication and mention of the grant of the patent: **11.06.2025**
(21) Application number: 21216691.2
(22) Date of filing: 21.12.2021
(51) Int. Cl.: C12Q 1/6806

(54) **METHODS AND KITS FOR DETECTING SPERM DNA FRAGMENTATION**
VERFAHREN UND KITS ZUM NACHWEIS DER DNA-FRAGMENTIERUNG VON SPERMIEN
PROCÉDÉS ET KITS DE DÉTECTION DE FRAGMENTATION D'ADN DU SPERME

(30) Priority: 23.12.2020 TW 109145792; 07.12.2021 US 202117643161
(43) Date of publication of application: 29.06.2022
(73) Proprietor: Bonraybio Co., Ltd., Taichung City 41280 (TW)
(72) Inventor: HSU, Cheng-Teng, 41280 Taichung City (TW); CHANG, Li-Sheng, 41280 Taichung City (TW); LEE, Hsiu-Chin, 41280 Taichung City (TW)
(74) Representative: Keltie LLP

(56) References cited:
- EP-A1- 1 710 320
- EP-A1- 2 637 019
- ENCISO M ET AL: "A new method to analyze boar sperm DNA fragmentation under bright-field or fluorescence microscopy", THERIOGENOLOGY, LOS ALTOS, CA, US, vol. 65, no. 2, 20 January 2006 (2006-01-20), pages 308 - 316, XP025076295, ISSN: 0093-691X, [retrieved on 20060120], DOI: 10.1016/J.THERIOGENOLOGY.2005.05.044
- CORTÉS-GUTIÉRREZ EI ET AL: "Assessment of Sperm DNA Fragmentation in Stallion ( Equus caballus ) and Donkey ( Equus asinus ) Using the Sperm Chromatin Dispersion Test", REPRODUCTION IN DOMESTIC ANIMALS, vol. 44, no. 5, 1 October 2009 (2009-10-01), DE, pages 823 - 828, XP055921085, ISSN: 0936-6768, DOI: 10.1111/j.1439-0531.2008.01091.x
- ANKEM M K ET AL: "Novel assay for determining DNA organization in human spermatozoa: Implications for male factor infertility", UROLOGY, BELLE MEAD, NJ, US, vol. 59, no. 4, 1 April 2002 (2002-04-01), pages 575 - 578, XP002343631, ISSN: 0090-4295, DOI: 10.1016/S0090-4295(01)01619-3
- PANNER SELVAM MANESH KUMAR ET AL: "A systematic review on sperm DNA fragmentation in male factor infertility: Laboratory assessment", ARAB JOURNAL OF UROLOGY, vol. 16, no. 1, 18 March 2018 (2018-03-18), pages 65 - 76, XP055919956, ISSN: 2090-598X, DOI: 10.1016/j.aju.2017.12.001

## Description

### FIELD

The present disclosure relates to a method for detecting the presence of sperm DNA fragmentation in a semen sample. The present disclosure also relates to a kit for detecting sperm DNA fragmentation in a semen sample.

### BACKGROUND

Sperm DNA integrity is crucial for embryo quality, embryo implantation, and embryo development. Sperm DNA fragmentation (SDF) can be caused by extrinsic factors, such as radiation, environmental pollutants, and chemotherapeutics, as well as intrinsic factors, such as defective spermatogenesis, sperm apoptosis, and oxidative stress. SDF may cause male infertility, failed *in vitro* fertilization (IVF), and miscarriage. Therefore, the detection of SDF is important for fertility testing and assisted reproductive techniques (ARTs).

Conventional methods for detecting SDF include sperm chromatin structure assay (SCSA), terminal deoxynucleotidyl transferase mediated dUTP nick end labeling (TUNEL) assay, DNA breakage detection-fluorescence in situ hybridization (DBD-FISH) test, comet assay (CA), and sperm chromatin dispersion (SCD) test.

Comet assay (CA), also known as single cell gel electrophoresis (SCGE), is a sensitive technique for detecting SDF. The procedures of CA involve embedding sperm cells in an agarose gel on a microscope slide, and then immersing the microscope slide in a lysis solution to break open the cell membrane and lyse the cellular proteins (e.g., protamine). Thereafter, the agarose gel is exposed to an electric field to attract negatively charged fragments of DNA toward the anode and form a comet-like structure. In the comet-like structure, the undamaged DNA nucleoid part is referred to as "head," and the trailing damaged DNA streak is referred to as "tail." After DNA staining with a fluorescent dye, the comet-like structure is visualized using a fluorescence microscope. Analysis of the comet tail can be performed by hand or with software, the fluorescence intensity of the comet tail indicating the extent of DNA damage. However, the operation of CA is complicated and time-consuming because of electrophoresis and software analysis processes, and thus CA cannot meet the needs of the industry.

SCD test is a modified halo assay that utilizes chemical methods to detect SDF. The procedures of the SCD test involve embedding sperm cells in an agarose gel, followed by DNA denaturation and deproteinization. Particularly, the double-stranded (DS) DNA of each sperm cell is denatured into a single-stranded (SS) DNA during the DNA denaturation. The nuclear protein (including protamine) of each sperm cell is lysed during the deproteinization. Therefore, DNA loops would be dispersed from the nuclear protein to the periphery of each sperm cell. After DNA staining with 4',6-diamidino-2-phenylindole (DAPI) or the Diff-Quik reagent, the dispersed DNA loops are monitored by fluorescence or brightfield microscopy. The DNA loops of the sperm cell with DNA fragmentation are smaller than that of the sperm cell without DNA fragmentation, and more difficult to be stained. More specifically, the head of the sperm cell without DNA fragmentation shows as a large halo (i.e., the halo width is at least one-third of the diameter of the core head of the sperm cell). In contrast, the head of the sperm cell with DNA fragmentation shows as a small halo or no halo (i.e., the halo width is smaller than one-third of the diameter of the core head of the sperm cell). However, determining the halo width is difficult.

There is a need to develop a method for rapid and accurate detection of SDF.

EP2637019 discloses a method comprising a step of detecting sperm DNA fragmentation by observing the formation of halos around the head of the sperm, wherein the cells are in a gel and wherein the gel may comprise e.g. alginate.

### SUMMARY

In a first aspect, the present disclosure provides a method according to claim 1 for detecting sperm DNA fragmentation (SDF) in a human semen sample. The method includes:
(a) embedding a semen sample containing sperm cells in a gel, which has a pore size from 3 to 9 nm and contains a component selected from the group consisting of acrylamide, acrylic acid, methacrylic acid, N-isopropylacrylamide (NIPAM) and alginate so as to obtain a sperm cells-embedded gel;
(b) treating the sperm cells-embedded gel with a lysis solution, to lyse the nuclear proteins of the sperm cells;
(c) subjecting the treated gel in step (b) to DNA staining; and
(d) observing the presence or absence of a halo formation around the heads of the sperm cells, wherein the presence of a halo formation is indicative of the presence of SDF.

In a second aspect, the present disclosure provides a method according to claim 2 for detecting SDF in a human semen sample. The method includes:
(a) embedding a semen sample containing sperm cells in a polyacrylamide gel containing acrylamide at a concentration ranging from 4% to 16% (w/v) with a ratio of acrylamide to bis-acrylamide ranging from 19:1 to 37.5:1 (w/w) to obtain a sperm cells-embedded polyacrylamide gel;
(b) treating the sperm cells-embedded polyacrylamide gel with a lysis solution to lyse nuclear proteins of the sperm cells;
(c) subjecting the treated polyacrylamide gel in step (b) to DNA staining; and
(d) observing the presence or absence of a halo formation around the heads of the sperm cells, wherein the presence of a halo formation is indicative of the presence of SDF.

In a third aspect, the present disclosure provides a kit according to claim 6 for detecting SDF in a semen sample.

The kit includes:
a gel-forming formulation comprising acrylamide in a concentration of 4-16% (w/v) with a ratio of acrylamide to bis-acrylamide ranging from 19:1 to 37.5:1 (w/ w)
a lysis solution; and
a DNA staining reagent.

### BRIEF DESCRIPTION OF THE DRAWINGS

Fig. 1 shows the morphological result of Example 1.
Fig. 2 shows the morphological observation result of each group of Example 2.
Fig. 3 shows a correlation plot between the DNA fragmentation index (DFI) determined according to the present method and the DFI determined according to comet assay (CA).
Fig. 4 is a schematic view illustrating the principle of the present method: in which integral DNA loops stay within small pores of the gel, and no halo formation is shown after DNA staining.
Fig. 5 is a schematic view illustrating the principle of the prior art method, where integral DNA loops are released and diffused from large pores of the gel, which causes a halo formation after DNA staining.

### DETAILED DESCRIPTION

The present disclosure provides a method for detecting sperm DNA fragmentation in a human semen sample, which includes:
(a) embedding the semen sample containing semen cells in a gel comprising acrylamide, acrylic acid, methacrylic acid, N-isopropylacrylamide (NIPAM) or alginate, wherein the gel has a pore size from 3 nm to 9 nm, so as to obtain a sperm cells-embedded gel;
(b) treating the sperm cells-embedded gel with a lysis solution comprising urea at a concentration ranging from 0.5 M to 4 M and sodium dodecyl sulfate (SDS) at a concentration ranging from 0.05% (w/v, g/mL) to 0.5% (w/v, g/mL), to lyse nuclear proteins of the sperm cells embedded in the gel;
(c) subjecting the treated gel in step (b) to DNA staining; and
(d) observing the presence or the absence of a halo formation around the heads of the sperm cells, wherein the presence of a halo formation is indicative of the presence of SDF.

In a preferred embodiment, the gel is a polyacrylamide gel.

According to the present disclosure, the polyacrylamide gel may be formed by reacting acrylamide with bis-acrylamide in the presence of an initiator.

In certain embodiments, a ratio of acrylamide to bis-acrylamide ranges from 19:1 (w/w) to 37.5:1 (w/w) .

According to the present disclosure, the initiator may be selected from the group consisting of ammonium persulfate (APS), N,N,N',N'-tetramethylethylenediamine (TEMED), riboflavin-5'-phosphate sodium, 3-(dimethylamino)propionitrile, and any combination thereof. In a preferred embodiment, the initiator is a combination of APS and TEMED.

According to the present disclosure, the semen sample may be collected from a male subject at any time. In one embodiment, the semen sample is collected from a male subject who has experienced sexual abstinence for at least 2 to 3 days but not greater than 10 days.

According to the present disclosure, the semen sample may be fresh or frozen (e.g., may be in a frozen form stored in liquid nitrogen (-196°C)).

As used herein, the term "subject" refers to any animal of interest, such as primates (e.g., humans, apes, and monkeys), non-primate mammals (e.g., pigs, cows, sheep, horses, goats, dogs, cats, mice, and rats), fish, and amphibians. In certain embodiments, the subject is a human.

According to the present disclosure, the semen sample may be diluted with a diluent to have a sperm concentration ranging from 4×10⁶ cells/mL to 2.8×10⁷ cells/mL.

Examples of the diluent may include, but are not limited to, Earle's medium, human tubal fluid (HTF) medium, tris-buffered saline (TBS), phosphate-buffered saline (PBS), and saline.

In certain embodiments, the semen sample is diluted with HTF medium to have a sperm concentration of 1×10⁷ cells/mL.

As used herein, the term "lysis solution" can be used interchangeably with the terms "cell lysis solution" and "protein lysis solution."

According to the present disclosure, in the lysis solution, sodium lauryl sulfate is used as an ionic surfactant, and urea is used as a protein denaturant. These two components can improve the lysis of protamine and thus the DNA loops can be easily released from the protamine to the periphery of the head of the sperm cell, and then be monitored as a halo via DNA staining, thereby reducing the time of lysis treatment (e.g., to less than 5 minutes).

According to the present disclosure, the lysis solution may further include an additional ionic or nonionic surfactant.

In certain embodiments, the additional ionic surfactant may be selected from the group consisting of sodium deoxycholate, sodium cholate, sodium lauroyl sarcosinate, and any combination thereof.

In certain embodiments, the additional nonionic surfactant may be selected from the group consisting of Triton X-100, Nonoxynol-40 (NP-40), Pluronic F-127 (F-127), Tween-20, and any combination thereof. In an exemplary embodiment, the additional nonionic surfactant is Triton X-100.

According to the present disclosure, the lysis solution may further include an additional protein denaturant. Examples of the additional protein denaturant may include, but are not limited to, 3-[(3-cholamidopropyl)dimethylammonio]-1-propanesulfonate hydrate, guanidinium chloride, and combinations thereof.

According to the present disclosure, the lysis solution may further include a reducing agent. Examples of the reducing agent may include, but are not limited to, dithiothreitol (DTT), β-mercaptoethanol, dithioerythritol (DTE), tributylphosphine (TBP), tris(2-carboxyethyl) phosphine (TCEP) hydrochloride, and combinations thereof. In an exemplary embodiment, the reducing agent is DTT.

According to the present disclosure, the lysis solution may further include salts. Examples of the salts may include, but are not limited to, sodium chloride (NaCl), potassium chloride (KCl), and combinations thereof.

According to the present disclosure, the lysis solution may further include a titrant. Examples of the titrant may include, but are not limited to, sodium hydroxide (NaOH), hydrochloric acid (HCl), and a combination thereof.

In certain embodiments, the lysis solution may further include 0.15 M to 3 M of NaCl, 0.05 M to 0.2 M of DTT, 0.1% (v/v) to 5% (v/v) of Triton X-100, and 0.01 M to 0.02 M of NaOH.

In an exemplary embodiment, the lysis solution includes 1 M urea, 0.05% (w/v, g/mL) of SDS, 2.5 M NaCl, 0.1 M DTT, 1% (v/v) of Triton X-100, and 0.02 M NaOH. In another exemplary embodiment, the lysis solution includes 4 M urea, 0.05% (w/v, g/mL) of SDS, 0.15 M NaCl, 0.2 M DTT, 0.5% (v/v) of Triton X-100, and 0.01 M NaOH. In yet another exemplary embodiment, the lysis solution includes 0.5 M urea, 0.5% (w/v, g/mL) of SDS, 3 M NaCl, 0.05 M DTT, 5% (v/v) of Triton X-100, and 0.015 M NaOH.

According to the present disclosure, the lysis solution may be adjusted to have a desired pH value. In certain embodiments, the lysis solution may have a pH value ranging from 7 to 9. In an exemplary embodiment, the lysis solution may have a pH value ranging from 7 to 8.2. In another exemplary embodiment, the lysis solution has a pH value of 7.5.

According to the present disclosure, the DNA staining is conducted using a staining method selected from the group consisting of Diff-Quik staining, Wright-Giemsa staining, propidium iodide (PI) staining, SYBR Green staining, DAPI staining, and acridine orange staining.

The present disclosure also provides a method for detecting SDF in a human semen sample, which includes:
(a) embedding the semen sample containing semen cells in a polyacrylamide gel containing acrylamide at a concentration ranging from 4% (w/v, g/mL) to 16% (w/v, g/mL) with a ratio of acrylamide to bis-acrylamide ranging from 19:1 to 37.5:1 (w/w), so as to obtain a sperm cells-embedded polyacrylamide gel;
(b) subjecting the sperm cells-embedded polyacrylamide gel to a lysis treatment with a lysis solution including urea at a concentration ranging from 0.5 M to 4 M and SDS at a concentration ranging from 0.05% (w/v, g/mL) to 0.5% (w/v, g/mL), so that nuclear proteins of the sperm cells embedded in the polyacrylamide gel are lysed;
(c) subjecting the treated polyacrylamide gel in step (b) to DNA staining; and
(d) observing the presence or the absence of a halo formation around the heads of the sperm cells, wherein the presence of a halo formation is indicative of the presence of SDF.

According to the present disclosure, in step (a), the polyacrylamide gel may have a pore size ranging from 3 nm to 9 nm.

The details of the operating conditions and reagents (i.e., the preparation of the semen sample, the ratio of acrylamide to bis-acrylamide, the initiator, the lysis solution, the DNA staining method, etc.) of this method are generally the same as those described above.

The present methods are easier to operate than the conventional tests, such as SCD and CA tests. Particularly, the person conducting the test only needs to identify the presence of a halo formation in the present methods, rather than to estimate the halo width in conventional tests.

Moreover, the present disclosure provides a kit for detecting sperm DNA fragmentation in a semen sample according to claim 6. The kit may include
a gel-forming formulation comprising acrylamide in a concentration ranging from 4-16% (w/ v) with a ratio of acrylamide to bis-acrylamide ranging from 19:1 to 37.5:1 (w/w)
a lysis solution including urea at a concentration ranging from 0.5 M to 4 M and SDS at a concentration ranging from 0.05% (w/v, g/mL) to 0.5% (w/v, g/mL); and
a DNA staining reagent.

According to the present disclosure, the gel-forming formulation may further include an initiator as described above.

In certain embodiments, acrylamide and the initiator are placed in separate containers (e.g., microcentrifuge tubes, glass bottles, or plastic bottles).

According to the present disclosure, the kit may further include a solid support for carrying the semen sample. The solid support includes a support base and an agarose layer disposed on a surface of the support base, and the agarose layer has an agarose concentration ranging from 0.25% (w/v, g/L) to 1.5% (w/v, g/L).

Examples of the support base may include, but are not limited to, a microscope slide and a well-plate.

In an exemplary embodiment, the support base is a microscope slide, and a surface of the microscope slide has been overlaid with a layer of 1% (w/v, g/L) agarose.

The detail of the lysis solution applied in this kit is generally the same as that described above.

According to the present disclosure, the DNA staining reagent may be selected from the group consisting of Diff-Quik solution, Wright-Giemsa solution, PI, SYBR Green, DAPI, and acridine orange.

The disclosure will be further illustrated by way of the following examples, which are intended for the purpose of illustration and should not be construed as limiting the disclosure in practice.

### EXAMPLES

### General Experimental Material: Lysis Solution

The lysis solution used in the following experiments contained 2.5 M NaCl, 0.2 M DTT, 4 M urea, 1% Triton X-100, 0.5% SDS, and 0.005 M sodium hydroxide (NaOH), and had a pH value ranging from 7.5 to 8.2.

### Example 1. Detecting sperm DNA fragmentation (SDF) by the present method.

### Experimental Procedures:

A semen sample without SDF of male Subject 1 (age between 22-40 years old) was collected, followed by liquefaction at room temperature. 30 µL of the liquefied semen sample was subjected to determination of the number of sperm cells using a semen quality analyzer (X1 PRO, LensHooke) in accordance with the manufacturer's instructions. Afterwards, a suitable amount of HTF medium was added to dilute the semen sample to reach sperm cell concentration from 0.07×10⁵ cells/µL to 0.28×10⁵ cells/µL. Two aliquots (70 µL each) of the diluted semen suspension of Subject 1 were used for the following experiments.

One aliquot served as an experiment sample, and the other aliquot served as a DNase-treated sample. Each aliquot was added with 69.2 µL of a 30% (w/v, g/mL) acrylamide/bis-acrylamide solution (Bio-Rad) and 30.8 µL of 0.01 M phosphate-buffered saline (PBS), followed by mixing with 1.5 µL of 10% *ammonium persulfate* (APS) and 1.5 µL of N,N,N',N'-tetramethylethylenediamine (TEMED). 20 µL of the respective resultant mixture was placed on an agarose layer (containing 1% (w/v, g/L) agarose) disposed on a surface of a microscope slide, followed by being left standing at room temperature for 3 to 5 minutes, such that the sperm cells were embedded in a polyacrylamide gel containing 12% (w/v, g/mL) acrylamide and were immobilized on the microscope slide. The resultant sperm cells-embedded polyacrylamide gel (PAG) was subjected to the following DNA hydrolysis treatment and is referred to as "sperm cells-PAG" hereinafter.

The sperm cells-PAG of the DNase-treated sample was treated with 0.1% Triton X-100, and was then washed with water twice for about 3 minutes, followed by conducting a DNA hydrolysis treatment using 2 U endonuclease DNase I (Cat. No. E1010, Zymo Research) for 30 minutes, to fragment the sperm DNA thereof. However, the sperm cells-PAG of the experiment sample received no such treatment and the sperm DNA is not fragmented.

Thereafter, about 200-300 µL of a lysis solution as described in General Experimental Material was added to the sperm cells-PAG of each of the experiment sample and the DNase-treated sample at room temperature for about 5-20 minutes. After washing with water two times, the lysed sperm cells-PAG was subjected to Diff-Quik staining for 1 minute using a DNA staining protocol well-known to those skilled in the art. The stained sperm cells-PAG was then observed and photographed under an optical microscope (BX-53, Olympus) at 100x and 200x magnifications.

### Results:

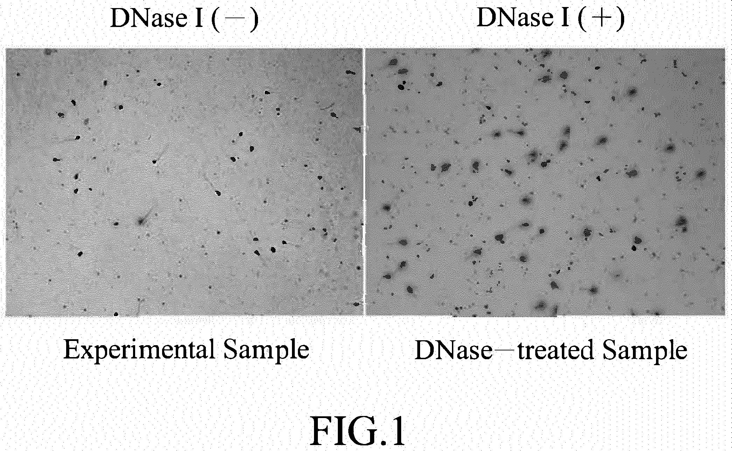

Referring to FIG. 1, halo formation (i.e., pink halos) was observed on the lysed sperm cells of the DNase-treated sample, while no halo formation was observed on the lysed sperm of the experiment sample.

In another experiment, endonuclease Alu I (Cat. No. R0137S, NEB) was used to replace endonuclease DNase I, and similar results were observed (data not shown).

These results show that the present method effectively detected sperm DNA fragmentation in a semen sample and is referred to as "sperm DNA fragment release (SDFR) assay" hereinafter.

### Example 2. Comparison of detecting SDF by the present method and by conventional methods.

### Experimental Procedures:

A semen sample of male Subject 2, age between 22-40 years old, was collected. Three aliquots were prepared according to Example 1. One aliquot served as an experimental sample detected by the present method, and the other two aliquots served as comparative samples 1 and 2, detected by comparison methods.

The SDF of the experimental sample was detected according to the SDFR method described in Example 1.

The SDF of comparative sample 1 was detected using the SCD test according to the following operating procedures. First, comparative sample 1 was mixed with 0.7% (w/v, g/mL) liquefied low melting agarose gel (Alfa Aesar) (in PBS). The mixture was placed on an agarose layer (containing 1% (w/v, g/L) agarose) disposed on a surface of a microscope slide at 4°C for 5 minutes, such that the sperm cells were embedded in the low melting agarose gel and were immobilized on the microscope slide. The resultant sperm cells-embedding agarose gel (AG) is referred to as "sperm cells-AG" hereinafter.

Thereafter, about 200-300 µL of a denaturing solution containing 0.1 N HCl was added to the sperm cells-AG of the comparative sample 1 at room temperature for about 7 minutes. The denatured sperm cells-AG was then treated with about 200-300 µL of a lysis solution as described in General Experimental Material at room temperature for about 5-20 minutes.

After washing with water two times, the lysed sperm cells-AG was subjected to Diff-Quik staining for 1 minute using a staining protocol well-known to those skilled in the art. The stained sperm cells-AG was then observed and photographed under an optical microscope (BX-53, Olympus) at 100x and 200x magnifications.

In addition, comparative sample 2 was subjected to detection of SDF using CA which was performed similar to the operating procedures of the SCD test described above, except that: the sperm cells-AG was not subjected to DNA denaturation treatment; and before DNA staining, the lysed sperm cells-AG was subjected to electrophoresis for about 20 minutes, followed by washing with 0.01 M PBS (pH 7.4) for about 5 minutes.

The procedures of the above-mentioned 3 different processes are summarized in Table 1.

**Table 1**

| | Gel | DNA Denaturation | Lysis treatment | Electrophoresis | DNA staining | Observation With SDF | Observation without SDF |
|---|---|---|---|---|---|---|---|
| experimental sample (the present SDFR test) | PAG | - | + | - | + | Presence of a halo | No halo |
| comparative sample 1 (SCD test) | Agarose | + | + | - | + | No halo or the halo width is smaller than 1/3 of the diameter of the core of nucleoid | The halo width is larger than 1/3 of the diameter of the core of nucleoid |
| comparative sample 2 (CA test) | Agarose | - | + | + | + | Presence of a comet tail | Absence of a comet tail |

The DFI(%) of each sample was calculated using technology known to those skilled in the art.

### Results:

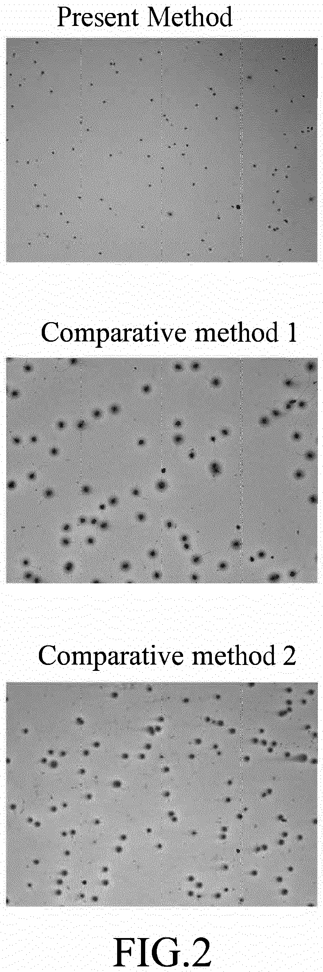

Referring to FIG. 2, in the experimental sample, SDF was detected by easily observing the presence or absence of halo formation around the sperm head. The results of the experiment sample by the present method show that Subject 2 has no SDF. In contrast to the present method, in the comparative sample 1, SDF was detected based on comparison of the size of the halo; while in the comparative sample 2, SDF was detected by observing the presence or absence of a comet tail. Although the DFIs determined in the experimental sample and the comparative samples 1 to 2 were similar (DFI = about 7%), it is not easy to detect SDF under SCD test and CA test due to dazzled and unclear halos in those tests.

These results show that the present method (i.e., the SDFR assay) requires fewer operating steps (SDFR dispenses with DNA denaturation treatment and electrophoresis), detects SDF quickly, and has a similar detection effect.

In addition, another aliquot of the semen sample of Subject 2 was placed in a cryoprotectant solution, and was then stored in liquid nitrogen for 72 hours in accordance with the fifth edition of the WHO laboratory manual for the examination and processing of human semen. Thereafter, the frozen semen sample was thawed at room temperature, and then subjected to determination of SDF according to the method described in Example 1. The DFI determined in the frozen semen sample was similar to that determined in the experimental sample (data not shown). The results indicate that the present method can effectively detect SDF in a frozen semen sample.

### Example 3. Evaluating accuracy of the present method.

### Experimental Procedures:

Fourteen semen samples from male Subjects (age between 22-40 years old) were collected and diluted to semen suspensions according to Example 1. The diluted semen suspensions were subjected to detection of SDF according to the present method similar to that performed for the experimental sample described in Example 1, and the DFI of the respective semen sample was calculated using technology known to those skilled in the art.

In addition, the 14 semen samples were also subjected to CA, and the operating procedures of the CA were similar to those described in Example 2, except that: a polyacrylamide gel was used to embed the semen sample instead, and ImageJ software was used to quantify the number of sperm cells with comet tails, so as to calculate DFI.

The DFIs, respectively determined based on the present method and the CA method, were then analyzed using linear regression and Pearson's correlation analysis to determine the correlation therebetween, and a coefficient of determination (R² value) was calculated.

### Results:

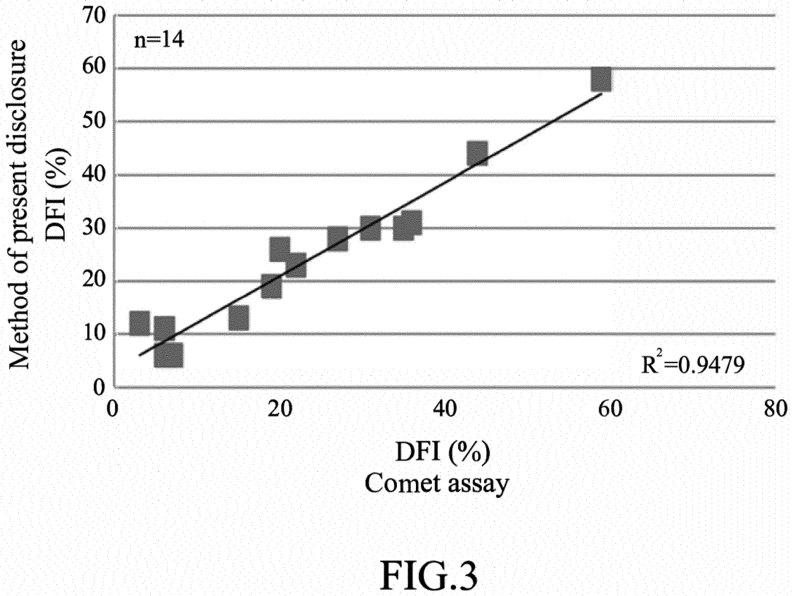

Referring to FIG. 3, the DFI determined according to the present method and the DFI determined according to the CA had an excellent correlation, with calculated R² value of 0.9479. The result indicates that the accuracy of the method of the present disclosure is similar to that of the CA.

### Example 4. Evaluating polyacrylamide gels with different acrylamide concentrations on the detection efficiency.

### Experimental Procedures:

A semen sample of male Subject 4 (age between 22-40 years old) was collected and was treated with 0.1% Triton X-100. The semen sample was then washed with water twice for about 3 minutes, followed by conducting a DNA hydrolysis treatment using 2 U endonuclease DNase I (Cat. No. E1010, Zymo Research) for 30 minutes to fragment the sperm DNA thereof. Portions of the hydrolyzed semen sample were used as 8 test samples (i.e., test samples 1 to 8).

The respective test sample was sequentially subjected to an embedding process, a lysis treatment, and DNA staining generally according to the procedures described in Example 1, except that the conditions shown in Table 2 were used for the embedding process.

**Table 2**

| Test sample | Semen sample (µL) | 30% (w/v, g/mL) Acrylamide/bis-acrylamide solution (µL) | PBS (µL) | APS (µL) | TEMED (µL) | Sperm cell concentration (cells/µL) | Final Polyacrylamide concentration in gel(%) (w/v, g/mL) |
|---|---|---|---|---|---|---|---|
| 1 | 70 | 23.1 | 76.9 | 1.5 | 1.5 | 0.1×10⁵ | 4 |
| 2 | 70 | 40.4 | 59.6 | 1.5 | 1.5 | 0.1×10⁵ | 7 |
| 3 | 70 | 57.7 | 42.3 | 1.5 | 1.5 | 0.1×10⁵ | 10 |
| 4 | 70 | 75.0 | 25 | 1.5 | 1.5 | 0.1×10⁵ | 13 |
| 5 | 70 | 92.3 | 7.7 | 1.5 | 1.5 | 0.1×10⁵ | 16 |
| 6 | 35 | 109.6 | 25.4 | 1.5 | 1.5 | 0.2×10⁵ | 19 |
| 7 | 35 | 126.9 | 8.1 | 1.5 | 1.5 | 0.2×10⁵ | 22 |
| 8 | 25 | 144.2 | 0.8 | 1.5 | 1.5 | 0.28×10⁵ | 25 |

The sperm cell concentration of each sample is defined by preliminary dilution. The sperm cell number of each sample needs to be constant. The DFI of each test sample was calculated using technology known to those skilled in the art.

### Results:

As shown in Table 3 below, the DFIs determined in test samples 1 to 7 were similar. The result indicates that the polyacrylamide gel containing acrylamide at a concentration ranging from 4% (w/v, g/mL) to 22% (w/v, g/mL) can be used in the present method.

**Table 3**

| Test sample | Acrylamide concentration (%) (w/v, g/mL) | DFI (%) |
|---|---|---|
| 1 | 4 | 81 |
| 2 | 7 | 84 |
| 3 | 10 | 84 |
| 4 | 13 | 82 |
| 5 | 16 | 82 |
| 6 | 19 | 85 |
| 7 | 22 | 85 |
| 8 | 25 | 55 |

The polyacrylamide gel containing acrylamide at a concentration ranging from 4% to 22% (w/v, g/mL) is calculated to have a pore size ranging from 8.8 nm to 3.2 nm (see B.M.A. Carvalho, et al. (2014), Sep. Purif. Rev., 43:241-262). In contrast, the AG under the SCD or CA test is calculated to have a pore size ranging from 70 nm to 600 nm (see Janaky Narayanan, et al. (2006), J. Phys. Conf. Ser., 28:83-86).

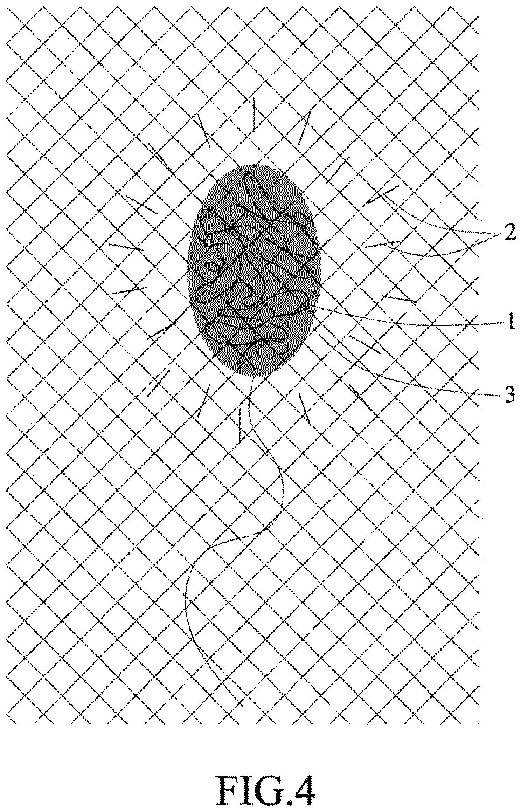

The results show that when a polyacrylamide gel having a pore size of 3.3 nm to 8.2 nm was used to embed the semen sample, followed by lysing the nuclear protein, the DNA loops without fragmentation (see the symbol "1" in Figure 4) did not diffuse from the sperm head (see the symbol "3" in Figure 4) because the volume of the DNA loop structure was larger than the pore size of the gel. Only DNA loops with fragmentation (see the symbol "2" in Figure 4) diffused from the sperm head and formed a halo. Therefore, the present method can directly detect SDF by visually observing whether there is a halo formed on the periphery of the sperm head.

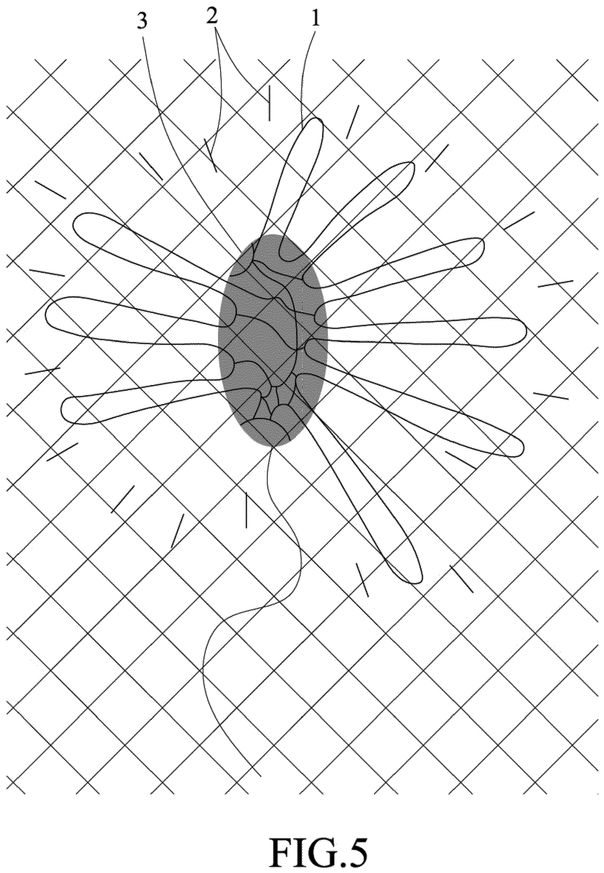

On the contrary, when a gel having a pore size of ≥ 70 nm (e.g., agarose gel) is used to embed the semen sample, followed by lysing the nuclear protein, the DNA loops without fragmentation (see the symbol "1" in Figure 5) and the DNA loops with fragmentation (see the symbol "2" in Figure 5) would all diffuse from the sperm head (see the symbol "3" in Figure 5), and therefore, it is not possible to detect SDF directly by visually observing whether there is a halo formed on the periphery of the sperm head, because there is too much halo noise from the DNA loops without fragmentation.

The invention, and the manner and process of making and using it, are now described in such full, clear, concise, and exact terms as to enable any person skilled in the art to which it pertains, to make and use the same. It is to be understood that the foregoing describes preferred embodiments of the present invention and that modifications may be made therein without departing from the scope of the present invention as set forth in the claims. To particularly point out and distinctly claim the subject matter regarded as invention, the following claims conclude the specification.

## Claims

1. A method for detecting sperm DNA fragmentation (SDF) in a semen sample from a human subject, comprising:
(a) embedding the semen sample containing sperm cells in a gel comprising acrylamide, acrylic acid, methacrylic acid, N-isopropylacrylamide (NIPAM), or alginate, to obtain a sperm cells-embedded gel, wherein the gel has a pore size from 3 nm to 9 nm;
(b) treating the sperm cells-embedded gel with a lysis solution to lyse the nuclear proteins of the sperm cells embedded in the gel;
(c) subjecting the treated gel in step (b) to DNA staining; and
(d) observing the presence or the absence of a halo formation around a head of each sperm cell, wherein the presence of halo formation is indicative of the presence of SDF.

2. A method for detecting SDF in a human semen sample, comprising:
(a) embedding the human semen sample containing sperm cells in a polyacrylamide gel containing acrylamide at a concentration ranging from 4% (w/v) to 16% (w/v), so as to obtain a sperm cells-embedded polyacrylamide gel, wherein the polyacrylamide gel is formed from acrylamide and bis-acrylamide in a ratio of acrylamide to bis-acrylamide ranging from 19:1 (w/w) to 37.5:1 (w/w);
(b) treating the sperm cells-embedding polyacrylamide gel with a lysis solution to lyse nuclear proteins of the sperm cells;
(c) subjecting the treated polyacrylamide gel in step (b) to DNA staining; and
(d) observing the presence or the absence of halo formation around a head of each sperm cell, wherein the presence of a halo formation is indicative of the presence of SDF.

3. The method of Claim 1 or 2, wherein the lysis solution includes a protein denaturant selected from the group consisting of urea, 3-[(3-cholamidopropyl)dimethylammonio]-1-propanesulfonate hydrate, guanidinium chloride, and combinations thereof.

4. The method of Claim 1 or 2, wherein the lysis solution includes an ionic surfactant selected from the group consisting of SDS, sodium deoxycholate, sodium cholate, sodium lauroyl sarcosinate, and combinations thereof.

5. The method of Claim 1 or 2, wherein the DNA staining is conducted with a staining reagent selected from the group consisting of Diff-Quik staining, Wright-Giemsa staining, propidium iodide (PI) staining, SYBR Green staining, 4',6-diamidino-2-phenylindole (DAPI) staining, and acridine orange staining.

6. A kit for detecting SDF in a human semen sample, comprising:
a gel-forming formulation comprising acrylamide in a concentration ranging from 4-16% (w/v), wherein the gel is formed from acrylamide and bis-acrylamide in a ratio of acrylamide to bis-acrylamide ranging from 19:1 (w/w) to 37.5:1 (w/w);
a lysis solution; and
a DNA staining reagent.

7. The kit of Claim 6, including an initiator selected from the group consisting of ammonium persulfate (APS), N,N,N',N'-tetramethylethylenediamine (TEMED), riboflavin-5'-phosphate sodium, 3-(dimethylamino)propionitrile, and combinations thereof.

8. The kit of Claim 6, wherein the DNA staining reagent is selected from the group consisting of Diff-Quik solution, Wright-Giemsa solution, propidium iodide (PI), SYBR Green, 4',6-diamidino-2-phenylindole (DAPI), and acridine orange.

9. The kit of Claim 6, further comprising a solid support for carrying the semen sample, the solid support including a support base and an agarose layer disposed on a surface of the support base, the agarose layer having an agarose concentration ranging from 0.25% (w/v) to 1.5% (w/v).

10. The kit of Claim 6, wherein the lysis solution comprises 0.5-4 M urea and 0.05-0.5 % (w/v) sodium dodecyl sulfate (SDS).

## Patentansprüche

1. Ein Verfahren zum Nachweis der Spermien-DNA-Fragmentierung in einer Samenprobe von einem menschlichen Individuum, das Folgendes beinhaltet:
(a) Einbetten der Spermienzellen enthaltenden Samenprobe in einem Gel, das Acrylamid, Acrylsäure, Methacrylsäure, N-Isopropylacrylamid (NIPAM) oder Alginat beinhaltet, um ein Gel, in dem Spermienzellen eingebettet sind, zu erhalten, wobei das Gel eine Porengröße von 3 nm bis 9 nm aufweist;
(b) Behandeln des Gels, in dem Spermienzellen eingebettet sind, mit einer Lyselösung, um die Zellkernproteine der in dem Gel eingebetteten Spermienzellen zu lysieren;
(c) Unterziehen des in Schritt (b) behandelten Gels einer DNA-Färbung; und
(d) Beobachten des Vorhandenseins oder der Abwesenheit einer Halobildung um einen Kopf jeder Spermienzelle, wobei das Vorhandensein einer Halobildung das Vorhandensein von SDF anzeigt.

2. Ein Verfahren zum Nachweis von SDF in einer menschlichen Samenprobe, das Folgendes beinhaltet:
(a) Einbetten der menschlichen Samenprobe, die Spermienzellen in einem Polyacrylamidgel enthält, das Acrylamid in einer Konzentration im Bereich von 4% (Gew./Vol.) bis 16% (Gew./Vol.) enthält, um somit ein Polyamidgel, in dem Spermienzellen eingebettet sind, zu erhalten, wobei das Polyacrylamidgel aus Acrylamid und Bisacrylamid in einem Verhältnis von Acrylamid zu Bisacrylamid im Bereich von 19:1 (Gew./Gew.) bis 37,5:1 (Gew./Gew.) gebildet ist;
(b) Behandeln des Polyacrylamidgels, in dem Spermienzellen eingebettet sind, mit einer Lyselösung, um die Zellkernproteine der Spermienzellen zu lysieren;
(c) Unterziehen des in Schritt (b) behandelten Polyacrylamidgels einer DNA-Färbung; und
(d) Beobachten des Vorhandenseins oder der Abwesenheit einer Halobildung um einen Kopf jeder Spermienzelle, wobei das Vorhandensein einer Halobildung das Vorhandensein von SDF anzeigt.

3. Verfahren gemäß Anspruch 1 oder 2, wobei die Lyselösung ein Proteindenaturierungsmittel umfasst, das aus der Gruppe ausgewählt ist, die aus Harnstoff, 3-[(3-Cholamidopropyl)dimethylammonio]-1-propansulfonathydrat, Guanidiniumchlorid und Kombinationen davon besteht.

4. Verfahren gemäß Anspruch 1 oder 2, wobei die Lyselösung ein ionisches Tensid umfasst, das aus der Gruppe ausgewählt ist, die aus SDS, Natriumdesoxycholat, Natriumcholat, Natriumlauroylsarcosinat und Kombinationen davon besteht.

5. Verfahren gemäß Anspruch 1 oder 2, wobei die DNA-Färbung mit einem Färbereagens durchgeführt wird, das aus der Gruppe ausgewählt ist, die aus Diff-Quik-Färbung, Wright-Giemsa-Färbung, Propidiumiodid(PI)-Färbung, SYBR-Green-Färbung, 4',6-Diamidino-2-phenylindol(DAPI)-Färbung und Acridinorange-Färbung besteht.

6. Ein Kit zum Nachweis von SDF in einer menschlichen Samenprobe, das Folgendes beinhaltet:
eine gelbildende Formulierung, die Acrylamid in einer Konzentration im Bereich von 4-16% (Gew./Vol.) beinhaltet, wobei das Gel aus Acrylamid und Bisacrylamid in einem Verhältnis von Acrylamid zu Bisacrylamid im Bereich von 19:1 (Gew./Gew.) bis 37,5:1 (Gew./Gew.) gebildet wird;
eine Lyselösung, und
ein DNA-Färbereagens.

7. Kit gemäß Anspruch 6, das einen Initiator umfasst, der aus der Gruppe ausgewählt ist, die aus Ammoniumpersulfat (APS), N,N,N',N'-Tetramethylethylendiamin (TEMED), Riboflavin-5'-phosphatnatrium, 3-(Dimethylamino)propionitril und Kombinationen davon besteht.

8. Kit gemäß Anspruch 6, wobei das DNA-Färbereagens aus der Gruppe ausgewählt ist, die aus Diff-Quik-Lösung, Wright-Giemsa-Lösung, Propidiumiodid (PI), SYBR Green, 4',6-Diamidino-2-phenylindol (DAPI) und Acridinorange besteht.

9. Kit gemäß Anspruch 6, das ferner einen festen Träger zum Tragen der Samenprobe beinhaltet, wobei der feste Träger eine Trägerbasis und eine auf der Oberfläche der Trägerbasis angeordnete Agaroseschicht umfasst, wobei die Agaroseschicht eine Agarosekonzentration im Bereich von 0,25% (Gew./Vol.) bis 1,5% (Gew./Vol.) aufweist.

10. Kit gemäß Anspruch 6, wobei die Lyselösung 0,5-4 M Harnstoff und 0,05-0,5 % (Gew./Vol.) Natriumdodecylsulfat (SDS) beinhaltet.

## Revendications

1. Procédé de détection de la fragmentation de l'ADN du sperme (SDF) dans un échantillon de sperme d'un sujet humain, comprenant :
(a) l'inclusion de l'échantillon de sperme contenant des spermatozoïdes dans un gel comprenant l'acrylamide, l'acide acrylique, l'acide méthacrylique, le N-isopropylacrylamide (NIPAM) ou l'alginate pour obtenir un gel comprenant des spermatozoïdes, le gel ayant une taille de pore de 3 nm à 9 nm ;
(b) le traitement du gel contenant des spermatozoïdes avec une solution de lyse pour lyser les protéines nucléaires des spermatozoïdes dans le gel ;
(c) la soumission du gel traité de l'étape (b) à une coloration de l'ADN ; et
(d) l'observation de la présence ou de l'absence d'une formation de halo autour d'une tête de chaque spermatozoïde, dans lequel la présence de la formation de halo indique la présence d'une SDF.

2. Procédé de détection d'une SDF dans un échantillon de sperme humain, comprenant :
(a) l'incorporation de l'échantillon de sperme humain contenant des spermatozoïdes dans un gel de polyacrylamide contenant de l'acrylamide à une concentration allant de 4 % (p/v) à 16 % (p/v), afin d'obtenir un gel de polyacrylamide contenant des spermatozoïdes, dans lequel le gel de polyacrylamide est formé à partir d'acrylamide et de bis-acrylamide dans un rapport de l'acrylamide au bis-acrylamide allant de 19:1 (p/p) à 37,5:1 (p/p) ;
(b) le traitement du gel de polyacrylamide contenant des spermatozoïdes avec une solution de lyse pour lyser les protéines nucléaires des spermatozoïdes ;
(c) la soumission du gel de polyacrylamide traité de l'étape (b) à une coloration de l'ADN ; et
(d) l'observation de la présence ou de l'absence d'une formation de halo autour d'une tête de chaque spermatozoïde, dans lequel la présence d'une formation de halo indique la présence d'une SDF.

3. Procédé selon la revendication 1 ou 2, dans lequel la solution de lyse comprend un dénaturant de protéine sélectionné dans le groupe constitué par l'urée, le 3-[(3-cholamidopropyl)diméthylammonio]-1-propanesulfonate hydrate, le chlorure de guanidinium ou des combinaisons de ceux-ci.

4. Procédé selon la revendication 1 ou 2, dans lequel la solution de lyse comprend un tensioactif ionique sélectionné dans le groupe constitué par le SDS, le désoxycholate de sodium, le cholate de sodium, le sarcosinate de lauroyle de sodium et des combinaisons de ceux-ci.

5. Procédé selon la revendication 1 ou 2, dans lequel la coloration de l'ADN est effectuée avec un réactif de coloration sélectionné dans le groupe constitué par la coloration Diff-Quik, la coloration Wright-Giemsa, la coloration d'iodure de propidium (PI), la coloration SYBR Green, la coloration 4',6-diamidino-2-phénylindole (DAPI) et la coloration orangé d'acridine.

6. Trousse de détection d'une SDF dans un échantillon de sperme humain, comprenant :
une formulation formant un gel comprenant de l'acrylamide à une concentration allant de 4 à 16 % (p/v), dans laquelle le gel est formé d'acrylamide et de bis-acrylamide dans un rapport de l'acrylamide au bis-acrylamide allant de 19:1 (p/p) à 37,5:1 (p/p) ;
une solution de lyse ; et
un réactif de coloration de l'ADN.

7. Trousse selon la revendication 6, comprenant un initiateur sélectionné dans le groupe constitué par le persulfate d'ammonium (APS), la N,N,N',N'-tétraméthylènediamine (TEMED), le riboflavin-5'-phosphate sodique, le 3-(diméthylamino)propionitrile et des combinaisons de ceux-ci.

8. Trousse selon la revendication 6, dans laquelle le réactif de coloration de l'ADN est sélectionné dans le groupe constitué par une solution Diff-Quik, une solution Wright-Giemsa, l'iodure de propidium (PI), le SYBR Green, le 4',6-diamidino-2-phénylindole (DAPI) et l'orangé d'acridine.

9. Trousse selon la revendication 6, comprenant en outre un support solide pour transporter l'échantillon de sperme, le support solide comprenant une base de support et une couche d'agarose disposée sur une surface de la base de support, la couche d'agarose ayant une concentration d'agarose allant de 0,25 % (p/v) à 1,5 % (p/v).

10. Trousse selon la revendication 6, dans laquelle la solution de lyse comprend de 0,5 à 4 M d'urée et de 0,05 à 0,5 % (p/v) de dodécyl sulfate de sodium (SDS).
